(19) **Europäisches Patentamt**

**European Patent Office**

**Office européen des brevets**

(11) **EP 0 827 950 B1**

(12) **EUROPEAN PATENT SPECIFICATION**

(45) Date of publication and mention
of the grant of the patent:
**27.11.2002 Bulletin 2002/48**

(51) Int Cl.$^7$: **C07C 231/02**

(21) Application number: **97306772.1**

(22) Date of filing: **02.09.1997**

(54) **Process for producing N-long-chain acyl acidic amino acids or salts thereof**

Verfahren zur Herstellung von mit N-(langkettigen Acylgruppen) substituierten sauren Aminosäuren

Procédé de préparation d'acides aminés acides N-acylés par un groupe à longue chaine, ou de leurs sels

(84) Designated Contracting States:
**DE FR GB IT**

(30) Priority: **06.09.1996 JP 25530396**

(43) Date of publication of application:
**11.03.1998 Bulletin 1998/11**

(73) Proprietor: **Ajinomoto Co., Inc.**
**Tokyo 104 (JP)**

(72) Inventors:
• **Hattori, Tatsuya, c/o Tokai Factory**
**Yokkaichi-shi, Mie-ken, 510-08 (JP)**
• **Hirai, Kiyomiki, c/o Tokai Factory**
**Yokkaichi-shi, Mie-ken, 510-08 (JP)**

(74) Representative: **Nicholls, Kathryn Margaret et al**
**MEWBURN ELLIS**
**York House**
**23 Kingsway**
**London WC2B 6HP (GB)**

(56) References cited:
   WO-A-91/12229       WO-A-94/27561
   DE-A- 2 449 445     GB-A- 1 483 500

• PATENT ABSTRACTS OF JAPAN vol. 017, no. 266 (C-1062), 25 May 1993 & JP 05 004952 A (MITSUBISHI PETROCHEM CO LTD), 14 January 1993,
• PATENT ABSTRACTS OF JAPAN vol. 095, no. 004, 31 May 1995 & JP 07 002747 A (HOECHST JAPAN LTD), 6 January 1995,

## EP 0 827 950 B1

**Description**

The present invention relates to a process for producing N-long-chain acyl acidic amino acids and in particular to a process for producing N-long-chain acyl acidic amino acids by reacting an acidic amino acid, glutamic acid or aspartic acid, or its salt with a long-chain fatty acid chloride.

It is known that inorganic salts (such as sodium, and potassium salts) as well as organic salts (such as triethanolamine salts) of N-long-chain acyl acidic amino acids (such as N-long-chain acyl glutamic acid or aspartic acid) have properties such as surface activating action, sterilizing action etc., and are thus used for various purposes, for instance as detergents, dispersants (i.e., dispersing agents), emulsifying agents, and anti-fungus agents (i.e., antibacterial agents).

A method of condensing glutamic acid with a long-chain fatty acid chloride in an aqueous solvent in the presence of an alkali is known as a method of synthesizing N-long-chain acyl acidic amino acids such as N-long-chain acyl glutamic acid (e.g., see the Reference Example at the begining of column 7 of Japanese Published Patent Application (kokai) No. 35058/1973). However, this prior art method has the problem that the yield of the object substance N-long-chain acyl glutamic acid is low relative to the long-chain fatty acid chloride. A similar method is disclosed at examples 1 and 2 of WO-A-94/27561.

One proposed improvement of the above method which is capable of producing the object N-long-chain acyl acidic acid efficiently and in which the reaction rate and reaction yield are improved and the reaction is completed in high yields in a short time, uses a tertiary amine or quaternary ammonium salt as a catalyst (Japanese Published Patent Application (kokai) No. 35058/1973 supra). However, in accordance with this method, the use of the catalyst entails problems such as increased facilities and process steps.

As an alternative method for increasing the yield, a method is known in which a mixed solvent of water and an organic solvent such as acetone, methyl ethyl ketone, dioxane, tetrahydrofuran or the like is used as the reaction solvent. Examples of such a method may be found in DE-A-24 49 445 and JP-A-5004952 but the use of such organic solvents causes problems in consideration of the influence of such solvents on the working environment and, particularly when safety is taken into consideration, the necessity for fulfillment of the regulations of the fire laws, and consequently the necessity for investment in facilities.

Therefore, in consideration of the necessity for improving the working environment worsened by the use of organic solvents, for maintaining safety at the time of manufacturing, and for investment in facilities to prevent the influence of organic solvents on the external environment, it is very much desired for N-long-chain acyl acidic amino acids to be produced in an aqueous solvent system without using any organic solvents.

Under the background of the above-mentioned prior art, an object of the present invention is to provide a method in which N-long-chain acyl acidic amino acid can be produced from an acidic amino acid and a long chain fatty acid chloride in the absence of the above-mentioned problems and in as high a yield as possible.

As a result of their eager research to achieve the above object, the present inventors have found that in a process for producing an N-long-chain acyl acidic amino acid by condensing an acidic amino acid and a long-chain fatty acid chloride in an aqueous solvent in the presence of an alkali, the problems of the prior art may be ameliorated by selecting suitable conditions for the condensation reaction, and on the basis of those findings, have completed of the present invention.

That is, the present invention relates to a process for producing an N-($C_8$-$C_{22}$ acyl) acidic amino acid or salt thereof, which comprises subjecting an acidic amino acid or a salt thereof and a C8 to C22 long-chain fatty acid chloride to condensation reaction in an aqueous solvent with no organic solvent present under stirring at a stirring power of not less than 0.2 kW/m$^3$ while keeping the pH in the range of 10 to 13 and the temperature in the range -10 to 30°.

Hereinafter, embodiments of the present invention will be described in detail.

The acidic amino acid or its salt used as one of the starting materials in the process of the present invention may be selected from glutamic acid and aspartic acid or salts thereof. These can be in the optically active form or racemic form without any particular limitation. As examples of such salts can be mentioned alkali metal salts such as the sodium salt, or the potassium salt.

The long-chain fatty acid chloride with which the acidic amino acid or its salt is to be condensed includes C8 to C22 straight chain or branched chain, saturated or unsaturated fatty acid chlorides. They can be single-component fatty acid chlorides such as oleoyl chloride, nonanoyl chloride, undecanoyl chloride, lauroyl chloride, tridecanoyl chloride, myristoyl chloride, stearoyl chloride and palmitoyl chloride. Further, various sorts of fatty acid chlorides such as mixed fatty acid chlorides such as palm oil fatty acid chloride, tallow fatty chloride, hardened tallow fatty chloride, soybean oil fatty acid chloride, cottonseed oil fatty acid chloride etc. can also be used similarly.

The base used for keeping the reaction system alkaline i.e. in the range of pH 10 to 13 is not particularly limited, and examples thereof are sodium hydroxide, potassium hydroxide, calcium hydroxide, sodium carbonate and ammonia, among which sodium hydroxide is particularly practical.

According to the present invention, an acidic amino acid or its salt and a long-chain fatty acid chloride are

2

allowed to react with each other in an aqueous solvent with stirring while the pH is maintained within a range of pH 10 to 13.

One of the major characterizing features of the present invention is that this stirring is carried out under strong stirring conditions at a stirring power of not less than $0.2$ kW/m$^3$, preferably not less than $0.3$ kW/m$^3$. By selecting such strong stirring conditions as the conditions for the condensation reaction, a yield as high as 80 % or more can be attained for the desired N-long-chain acyl acidic amino acid (see Table 1 below). Even though the same aqueous solvent is used in the Reference Example in Japanese Published Patent Application (kokai) No. 35058/1973 above, the yield therein was only 50 odd %.

The following numerical formula (1) is known as a formula for determining the amount of energy required for stirring (power (P) consumed for stirring) in a reaction vessel (see pages 505 and 553 in "Kakuhan Gijyutu" (Stirring Technology) edited by Satake Kagaku Kikai Kogyo K.K. and compiled under the supervision of Kazuo Yamamoto et al. (published in 1992 by Satake Kagaku Kikai Kogyo K.K.)).

$$P = NT/97376 \text{ (kW)} \qquad\qquad (1)$$

where N is the rotation number of a stirring shaft (r/min.), and T is the stirring torque (kgf·cm).

According to the present invention, the power consumed for stirring per unit volume of a reaction solution is newly defined as stirring power (Q) as an indicator of the stirred conditions in a reaction vessel, as in the numerical formula (2):

$$Q = NT/97376V \text{ (kW/m}^3\text{)} \qquad\qquad (2)$$

where V is the volume of a reaction solution (m$^3$).

As is evident from this definition, the stirring power is rendered independent on the volume of the reaction solution in the reaction vessel, and the stirring power can be used as an indicator of stirred conditions, and according to the present invention, as an indicator of reaction yields as well.

It is necessary that as the reaction proceeds, the viscosity of the reaction solution remains within a certain allowable range in order to use the stirring power (Q) as an indicator of the reaction yield according to the present invention. With respect to this, the change of electric current applied to a rotating blade on a bench-plant scale was determined every 10 minutes over 3 hours and 45 minutes from the initiation until the termination of the reaction. The applied electric current was 5.7 A (ampere) just after the initiation of the reaction, and it was 5.5 A at the time of termination of the reaction, and even during the reaction, it was almost constant in the range of 5.2 to 5.6 A. Even in the actual viscosity measurement of the reaction system according to the method of the present invention, the viscosity was 10 cp or thereabout before the reaction, and it was about 50 cp or thereabout after the reaction (as determined at 20°C with a Brookfield type viscometer), so it is not necessary to take into consideration increase in stirring torque due to increase in viscosity.

The reaction temperature according to the present invention is not particularly limited, and the condensation reaction proceeds in a wide range of, e.g., about -20°C to about 50°C. To attain a high yield, e.g., a high yield as high as 80 % or more, however, the temperature is to be maintained in the range of -10°C to 30°C, more preferably 5°C to 30°C, and a lower temperature within this range will bring about a higher yield of the object substance.

With respect to the ratio of the acidic amino acid or its salt to the long-chain fatty acid chloride, it is preferable to use the former in an at least equimolar amount relative to the latter in order to raise the yield of the object substance, and a higher ratio of the former within this range will bring about a higher yield.

The yield mentioned herein is the yield of the N-long-chain acyl acidic amino acid or its salt relative to (i.e., on the basis of) the long-chain fatty acid chloride.

To attain a high yield, e.g., 80 % or more, the ratio (molar ratio) of the acidic amino acid or its salt to the long-chain fatty acid chloride can be within the range of 1.1 to 1.5 for practical purposes.

If the initial concentration of an acidic amino acid or its salt at the start of the reaction is 25 % or more, the yield of the object substance will exceed 80 %, and a 53 % yield will be brought about by an initial concentration of 19 %, a 78 % yield by 20 %, and a 83 % yield by 30 %. For a high yield of the object substance N-long-chain acyl acidic amino acid or its salt, therefore, the initial concentration of an acidic amino acid or its salt is preferably within the range of 20 to 60 %, more preferably 25 to 50 % to achieve a high yield of 80 % or more.

After the reaction is completed, if the reaction mixture is made acidic with a mineral acid such as sulfuric acid, hydrochloric acid or the like, crude crystals of the N-long-chain acyl acidic amino acid are precipitated. Such crude crystals after they are separated, e.g., by filtering are washed in a usual manner with an alkane solvent such as petroleum benzine whereby the N-long-chain acyl acidic amino acid can be obtained in high yields. On the other hand, such crystals after they are separated can be neutralized with an aqueous NaOH solution, an aqueous KOH solution, an aqueous triethanolamine solution or the like to give the corresponding N-long-chain acyl acidic amino acid salt.

Examples:

Hereinafter, embodiments of the present invention are described in more detail by reference to Examples.

Example 1. (Synthesis of N-lauroyl-L-glutamic acid/laboratory scale (1))

50 g (0.27 mol) of monosodium L-glutamate was suspended in 60 ml water and 41.5 g of aqueous 25 % sodium hydroxide solution was added thereto to prepare an aqueous solution with a pH of 12.0. While this solution was kept at 20°C with stirring at a stirring power of 0.44 kW/m$^3$, 45.0 g (0.20 mol) of lauric acid chloride and 18.8g of aqueous 25 % sodium hydroxide solution were simultaneously added dropwise to the solution over a period of 1 hour during which the pH and temperature were maintained at the values described above. After this dropwise addition was finished, the mixture was stirred for additional 1.5 hours, and 13.9 g of aqueous 25 % sodium hydroxide solution was consumed to complete the reaction.

The reaction mixture was adjusted to pH 1 with 15 % sulfuric acid, and cold water was introduced into it to precipitate crystals. The weight of the crystals separated by centrifugation was 62 g. From the result of their analysis by HPLC, it was found that the yield of the N-lauroyl-L-glutamic acid was 83 % (based on the lauric acid chloride). In this connection, the HPLC made use of an ODS column and methanol/a pH 3 phosphate buffer (a 75/25 mixture) as an eluent (detection at 210 nm at a temperature of 60°C).

The same experiment was repeated where the stirring power was varied as shown in Table 1 below. The results (yields) are also shown in the same table. For reference, the number of revolutions is also shown.

Example 2. (Synthesis of N-lauroyl-L-glutamic acid/laboratory scale (2))

50 g (0.27 mol) of monosodium L-glutamate was suspended in 60 ml water and 41.5 g of aqueous 25 % sodium hydroxide solution was added thereto to prepare an aqueous solution with a pH of 12.0. While this solution was kept at 10°C with stirring with a homogenizer (stirring power: 25.0 kW/m$^3$), 45.0 g (0.20 mol) of lauric acid chloride and 33.5 g of aqueous 25 % sodium hydroxide solution were simultaneously dropped into the solution over a period of 2 hours during which the pH and temperature were maintained at the values described above. After this dropwise addition was finished, the mixture was stirred for additional 0.5 hour, and 2.1 g of aqueous 25 % sodium hydroxide solution was consumed to complete the reaction.

The reaction mixture was adjusted to pH 1 with 15 % sulfuric acid and cold water was added to it to precipitate crystals. The weight of the crystals separated by centrifugation was 65 g. The result of their analysis in the same manner as in Example 1 indicated that the yield of the N-lauroyl-L-glutamic acid was 87 %.

Example 3. (Synthesis of N-cocoyl-L-glutamic acid/commercial plant scale (1))

800 kg (4.3 kilomol) of monosodium L-glutamate was suspended in 975 L water and 800 kg of aqueous 25 % sodium hydroxide solution was added to it to prepare an aqueous solution at with a pH of 12.0. While this solution was kept at 20°C with stirring at a stirring power of 0.61 kW/m$^3$, 783 kg (3.3 kilomol) of cocoyl chloride and 542 kg of aqueous 25 % sodium hydroxide solution were simultaneously dropped into the solution over a period of 3 hours during which the pH and temperature were maintained at the values described above. After this dropwise addition was finished, the mixture was stirred for additional 1.5 hours, and 117 kg of aqueous 25 % sodium hydroxide solution was consumed to complete the reaction.

The reaction mixture was adjusted to pH 1 with 15 % sulfuric acid and cold water was added to it to precipitate crystals. The weight of the crystals separated by centrifugation was 975 kg. The result of their analysis in the same manner as in Example 1 indicated that the yield of the N-cocoyl-L-glutamic acid was 80 %.

Example 4. (Synthesis of N-cocoyl-L-glutamic acid/bench plant scale (1))

22.6 kg (121 mol) of monosodium L-glutamate was suspended in 27.5 L water and 18.9 kg of aqueous 25 % sodium hydroxide solution was added to it to prepare an aqueous solution with a pH of 12.0. While this solution was kept at 20°C with stirring at a stirring power of 0.70 kW/m$^3$, 20.8 kg (93 mol) of cocoyl chloride and 15.3 kg of aqueous 25 % sodium hydroxide solution were simultaneously dropped into the solution over a period of 2 hours during which the pH and temperature were maintained at the values described above. After this dropwise addition was finished, the mixture was stirred for additional 1.0 hour, and 3.3 kg of aqueous 25 % sodium hydroxide solution was consumed to complete the reaction.

The reaction mixture was adjusted to pH 1 with 15 % sulfuric acid and cold water was added to it to precipitate crystals. The weight of the crystals separated by centrifugation was 29 kg. The result of their analysis in the same manner as in Example 1 indicated that the yield of the N-cocoyl-L-glutamic acid was 83 %.

The results of Examples 1 to 4 are collectively shown in Table 1 below. In this table, Experiment Nos. L1a to L1c refer to Comparative Examples.

Table 1 :

| Relationship Between Stirring Power and Yield | | | |
|---|---|---|---|
| Experiment Number | Stirring Power (kW/m3) | Yield (%) | Revolution Number (rpm) |
| L1a | 0.003 | 33 | 50 |
| L1b | 0.03 | 40 | 100 |
| L1c | 0.09 | 51 | 150 |
| L1d | 0.22 | 75 | 200 |
| L1e | 0.30 | 81 | 220 |
| L1f | 0.44 | 83 | 250 |
| CP | 0.61 | 80 | 105 |
| L1g | 0.63 | 83 | 270 |
| BP | 0.70 | 83 | 277 |
| L1h | 0.71 | 82 | 300 |
| L1i | 0.91 | 83 | 350 |
| L1j | 1.00 | 84 | 360 |
| L1k | 1.47 | 84 | 400 |
| L1l | 2.71 | 84 | 500 |
| L1m | 3.29 | 84 | 550 |
| L2 | 25.0 | 87 | 20,000 |
| L1a~L1m : Laboratory scale (1) | | | |
| L2 : Laboratory scale (2) | | | |
| CP : Commercial Plant scale (1) | | | |
| BP : Bench plant scale (1) | | | |

Example 5. (Synthesis of N-cocoyl-L-aspartic acid)

50 g (0.38 mol) of L-aspartic acid was suspended in 45 ml water and 118g of aqueous 25 % sodium hydroxide solution was added to it to prepare an aqueous solution with a pH of 12.0. While this solution was kept at 20°C with stirring at a stirring power of 0.48 kW/m$^3$, 64.6 g (0.29 mol) of cocoyl chloride and 33 g of aqueous 25 % sodium hydroxide solution were simultaneously dropped into the solution over a period of 3 hours during which the pH and temperature were maintained at the values described above. After this dropwise addition was finished, the mixture was stirred for additional 1.5 hours, and 22 g of aqueous 25 % sodium hydroxide solution was consumed to complete the reaction.

The reaction mixture was adjusted to pH 1 with 15 % sulfuric acid and cold water was added to it to precipitate crystals. The crystals were recovered by filtration, and the result of their analysis by HPLC indicated that N-cocoyl-L-aspartic acid was obtained in an 80 % yield.

In conclusion, embodiments of the present invention may enable N-long-chain acyl acidic acids or salts thereof useful in facial washing agents, hair and body cleaning agents etc. to be easily obtained in high yields by reacting a long-chain fatty acid chloride with an acidic amino acid or its salt in an aqueous solution.

**Claims**

1. A process for producing an N-(C$_8$-C$_{22}$-acyl) acidic amino acid or salt thereof, which comprises subjecting an acidic amino acid or a salt thereof and a C8 to C22 long-chain fatty acid chloride to condensation reaction in an aqueous solvent with no organic solvent present with stirring at a stirring power of not less than 0.2 kW/m$^3$ while keeping the pH in the range of 10 to 13 and the temperature in the range -10 to 30°.

2. A process according to claim 1 wherein said stirring power is selected so that the yield of said acid or salt is at least 80% based on the fatty acid chloride.

3. a process according to claim 1 or claim 2 including a subsequent step of acidifying the reaction mixture with a mineral acid and separating the resulting crystals of the acylamino acid.

**Patentansprüche**

1. Verfahren zur Herstellung einer sauren N-(C$_8$-C$_{22}$-Acyl)aminosäure oder ihres Salzes, welches die Durchführung einer Kondensationsreaktion einer sauren Aminosäure oder ihres Salzes mit einem langkettigen C$_8$-C$_{22}$-Fettsäurechlorid in wäßriger Lösung in Abwesenheit eines organischen Lösungsmittels unter Rühren bei einer Rührleistung von nicht weniger als 0,2 kW/m$^3$ umfaßt, während der pH-Wert im Bereich von 10 bis 13 und die Temperatur im Bereich von -10 bis 30°C gehalten wird.

2. Verfahren nach Anspruch 1, wobei die genannte Rührleistung so gewählt wird, daß die Ausbeute der Säure oder ihres Salzes mindestens 80%, bezogen auf das Fettsäurechlorid, ist.

3. Verfahren nach Anspruch 1 oder 2, welches eine nachfolgende Stufe umfasst, bei der die Reaktionsmischung mit einer Mineralsäure angesäuert wird und die erhaltenen Acylaminosäurekristalle abgetrennt werden.

**Revendications**

1. Procédé pour produire un acide aminé acide N-acylé par un groupe en C$_8$-C$_{22}$ ou un sel de celui-ci, qui comprend de soumettre un acide aminé acide ou un sel de celui-ci et un chlorure d'acide gras à longue chaîne en C$_8$ à C$_{22}$ à une réaction de condensation dans un solvant aqueux avec aucun solvant organique présent en agitant à une puissance d'agitation qui n'est pas inférieure à 0,2 kW/m$^3$ tout en maintenant le pH dans l'intervalle compris entre 10 et 13 et la température dans l'intervalle compris entre -10 et 30°.

2. Procédé selon la revendication 1 dans lequel ladite puissance d'agitation est choisie de façon telle que le rendement dudit acide ou sel soit d'au moins 80 % sur la base du chlorure d'acide gras.

3. Procédé selon la revendication 1 ou la revendication 2 comprenant une étape subséquente consistant à acidifier le mélange de réaction avec un acide minéral et à séparer les cristaux résultants de l'acide aminé acylé.